## Europäisches Patentamt
## European Patent Office
## Office européen des brevets

(11) Veröffentlichungsnummer: **0 141 975**
**B1**

(12) **EUROPÄISCHE PATENTSCHRIFT**

(45) Veröffentlichungstag der Patentschrift:
27.04.88

(21) Anmeldenummer: **84111155.2**

(22) Anmeldetag: **19.09.84**

(51) Int. Cl.⁴: **C 07 C 69/14, C 07 C 67/08**

(54) Verfahren zur Veresterung von Essigsäure mit Alkoholen, deren Essigsäureester höher sieden als Essigsäure.

(30) Priorität: **12.10.83 DE 3337101**

(43) Veröffentlichungstag der Anmeldung:
**22.05.85 Patentblatt 85/21**

(45) Bekanntmachung des Hinweises auf die Patenterteilung:
**27.04.88 Patentblatt 88/17**

(84) Benannte Vertragsstaaten:
**BE DE FR GB IT NL**

(56) Entgegenhaltungen:
**EP-A-0 066 059**
**EP-A-0 105 111**
**DE-B-1 919 527**
**US-A-4 314 947**

(73) Patentinhaber: **HÜLS AKTIENGESELLSCHAFT, - RSP Patente / PB 15 - Postfach 13 20, D-4370 Marl 1 (DE)**

(72) Erfinder: **Oberholz, Alfred, Dr., Lipper Weg 197, D-4370 Marl (DE)**
Erfinder: **Böxkes, Werner, Dr., Oppauer Strasse 2, D-4370 Marl (DE)**

## Beschreibung

Die Erfindung betrifft ein Verfahren zur Gewinnung und Reinigung von Estern der Essigsäure mit Alkoholen, deren Essigsäureester höher sieden als Essigsäure.

Die Herstellung von Essigsäureestern durch Umsetzung der Säure mit Alkoholen in flüssiger Phase ist bekannt. Die Reaktion wird vorteilhaft unter $H^+$-Ionen-Katalyse durchgeführt, welche durch starke bis mittelstarke anorganische oder organische Säuren zur Verfügung gestellt werden können. Diese Verfahren weisen jedoch den Nachteil auf, daß wegen der Korrosivität der Reaktionsgemische Schäden an den verwendeten Apparaturen auftreten, wenn diese nicht aus kostspieligen, korrosionsbeständigen Materialien gefertigt sind. Außerdem muß der entstandene Ester durch Destillation von den flüssigen Katalysatoren abgetrennt werden, wobei insbesondere bei Etheralkoholen Nebenprodukte gebildet werden.

Diese Nachteile werden vermieden, wenn statt der flüssigen Säuren saure Ionenaustauscher als Katalysatoren verwendet werden. Mit dem Einsatz dieser Ionenaustauscher sind jedoch ebenfalls gravierende Nachteile verbunden (Chemie-Technik 11 (1959), Seiten 24 bis 26); zum einen erlaubt ihr Einsatz nur verhältnismäßig geringe Durchsätze, zum anderen werden in der mit Ionenaustauschern gefüllten Veresterungskolonne erhebliche Mengen der aufsteigenden Esterdämpfe wieder gespalten. Bei Etheralkoholen tritt zudem durch katalytisch geförderte Spaltung der Etherbindung eine Vielzahl, die Ausbeute herabsetzender Nebenprodukte auf.

Diese Nachteile vermeidet die DE-Patentanmeldung 32 35 531.9, nach der die Veresterung in einem Festbettreaktor bis zum Gleichgewicht geführt wird und die Reaktionsprodukte durch Destillation in einer Kolonne, beispielsweise einer Füllkörperkolonne anschließend in Rohester und Wasser sowie unumgesetzte Säure-Alkohol-Gemische aufgetrennt werden. Das Verfahren hat jedoch den Nachteil, daß die Umsetzung nur bis zum Veresterungsgleichgewicht geführt werden kann, wodurch es notwendig ist, große Mengen unumgesetzter Edukte vom Produkt destillativ abzutrennen. Die hierdurch erzwungene Kreisfahrweise von größeren Mengen an Säure und Alkohol ist kostenintensiv und hebt die Vorteile dieses Verfahrens gegenüber den zuvor beschriebenen Verfahren teilweise wieder auf.

Nach der EP-A-0 066 059 wird in einem Reaktor ebenfalls bis zum Gleichgewicht verestert, wobei die Lage des Gleichgewichts von den Einsatzstoffen bestimmt wird.

Im Falle der niedrig siedenden Ester werden aber Wasser, Ester und ein Teil der Alkohole entsprechend der vorgegebenen binären und ternären Azeotrope über Kopf der ersten Kolonne genommen. Hierbei ist die Säurefreiheit des Kopfproduktes wichtig, was auch bei Estern bzw.

Azeotropen mit Siedepunkten unterhalb der Essigsäure möglich ist, jedoch nicht bei Reaktionsgemischen, die bis auf das Wasser höher sieden als Essigsäure.

Der Erfindung liegt die Aufgabe zugrunde, diese Nachteile zu vermeiden bzw. zu vermindern ohne die verfahrenstechnischen Vorteile der Veresterung an Kationenaustauschern aufzugeben.

Diese Aufgabe wird erfindungsgemäß entsprechend den Angaben der Patentansprüche gelöst. Man setzt zunächst in einer ersten Stufe Essigsäure und Alkohol an einem festen stark sauren Kationenaustauscher bis zum Gleichgewicht um und verestert anschließend in einer zweiten Stufe im Abtriebsteil einer Kolonne bei Temperaturen oberhalb 100° C und Verweilzeiten von 40 bis 180 min weiter. Hierdurch gelingt es überraschenderweise, ohne Einwirkung eines Katalysators, erhebliche weitere Anteile an Essigsäure und Alkohol über das Veresterungsgleichgewicht hinaus umzusetzen, so daß weniger nicht umgesetzte Edukte im Kreis gefahren werden müssen.

Geeignete Alkohole sind beispielsweise n-Butanol, Pentanole, Hexanole, Heptanole, Octanole und Glykolether, wie Ethylenglykolmonoethylether, Diethylenglykolmonoethylether, Propylenglykolmonomethylether.

Das Molverhältnis Alkohol Essigsäure beträgt im allgemeinen 0,5 bis 10 : 1, vorzugsweise 1,0 bis 2,0 : 1.

In der ersten Stufe führt man die Veresterung bei einer Temperatur von 70 bis 140° C durch, bei den Alkoholen vorzugsweise bei einer Temperatur von 90 bis 120° C, bei den Glykolethern vorzugsweise bei einer Temperatur von 70 bis 90° C, in einem Druckbereich von 1,01 bis 10 bar, bei den Glykolethern vorzugsweise von 1,01 bis 2 bar.

Als Katalysatoren sind grundsätzlich alle stark sauren feinkörnigen Kationenaustauscher geeignet, deren Menge vorteilhaft so bemessen ist, daß dem Reaktionsgemisch, das den Reaktor in flüssiger Phase durchströmt, eine Verweilzeit von 2 bis 120 min, bei primäre Alkoholen vorzugsweise von 2 bis 15 min eingeräumt wird. Vor dem Einsatz läßt man den Kationenaustauscher in dem Essigsäure-Alkohol-Gemischt, das zur Veresterung eingesetzt wird, bis zur Volumenkonstanz quellen.

Als Reaktor setzt man eine vertikalen Reaktor ein, insbesondere einen Rohrreaktor, der den körnigen Katalysator enthält, der von einem feinmaschigen Sieb, einer porösen Platte oder einer ähnlichen, geeigneten Vorrichtung gehalten wird, und durch den das Reaktionsgemisch aufwärts oder abwärts, vorzugsweise abwärts, strömt.

Das Veresterungsgemisch fördert man anschließend in eine nachgeschaltete Rektifikationskolonne, die unter einem Kopfdruck von 1 bis 5 bar, vorzugsweise 1 bis 3 bar, bei Glykolethern vorzugsweise 1,0 bis 1,5 bar betrieben wird. Im Abtriebsteil der Kolonne führt man die Veresterung des in der ersten Stufe nicht umgesetzten Essigsäure-Alkohol-Gemisches bei einer Temperatur oberhalb 100°C, vorzugsweise 120 bis 240°C durch. Der Abtriebsteil der Kolonne weist einen Druckverlust von 2 bis 10 mbar, vorzugsweise 3 bis 8 mbar pro theoretischer Stufe auf. Beispielsweise enthält der Abtriebsteil Böden, z. B. Glockenböden oder Tunnelböden, die einen Druckverlust von 2 bis 10 mbar, vorzugsweise 2 bis 8 mbar/Boden aufweisen, der durch ein entsprechend hohes Ablaufwehr und dem damit verbundenen hohen Flüssigkeitsstand auf den Böden hervorgerufen wird. Die notwendige Wärme wird der Kolonne über einen Verdampfer, beispielsweise einen Umlaufverdampfer oder über eine Heizschlange zugeführt. Der Flüssigkeitsinhalt im Abtriebsteil einschließlich des Sumpfes ist so bemessen, daß das Produktengemisch in diesem Teil eine Verweilzeit von 40 bis 180 min, vorzugsweise 60 bis 120 min hat.

Das bei der Veresterung gebildete Reaktionswasser destilliert man mit einem Schleppmittel azeotrop über Kopf ab, wobei als Schleppmittel beispielsweise Toluol oder Cyclohexan oder bevorzugt auch der während der Reaktion gebildete Ester, wenn er mit dem Wasser allein oder mit Wasser und dem Alkohol ein Heteroazeotrop bildet, verwendet werden können. Die am Kolonnenkopf kondensierten Brüden trennt man in zwei Phasen. Die in der Hauptsache das Schleppmittel enthaltende Oberphase wird vollständig, die das Reaktionswasser enthaltende Unterphase teilweise auf den Kolonnenkopf zurückgegeben oder vollständig abgenommen.

Der Produktester wird gemeinsam mit den noch nicht umgesetzten Mengen an Essigsäure und Alkohol flüssig aus dem Sumpf abgezogen und in einer nachgeschalteten Rektifikationskolonne in Reinester und ein Gemisch aus Essigsäure und Alkohol, welches auch noch kleinere Mengen Ester und Wasser enthalten kann, aufgetrennt. Dieses Gemisch wird mit frischen Edukten entsprechend dem Umsatz ergänzt und wieder dem Reaktor · zugeführt. Die Reinesterabnahme am Sumpf der Reinesterkolonne erfolgt dampfförmig. Aus dem Sumpf können flüssig geringe Mengen höher siedender Produkte ausgeschleust werden.

Man erhält die Ester dem Essigsäure in einer Ausbeute, die erheblich über dem Veresterungsgleichgewicht liegt, beispielsweise 40 bis 70 % oberhalb des Veresterungsgleichgewichts.

## Beispiel 1

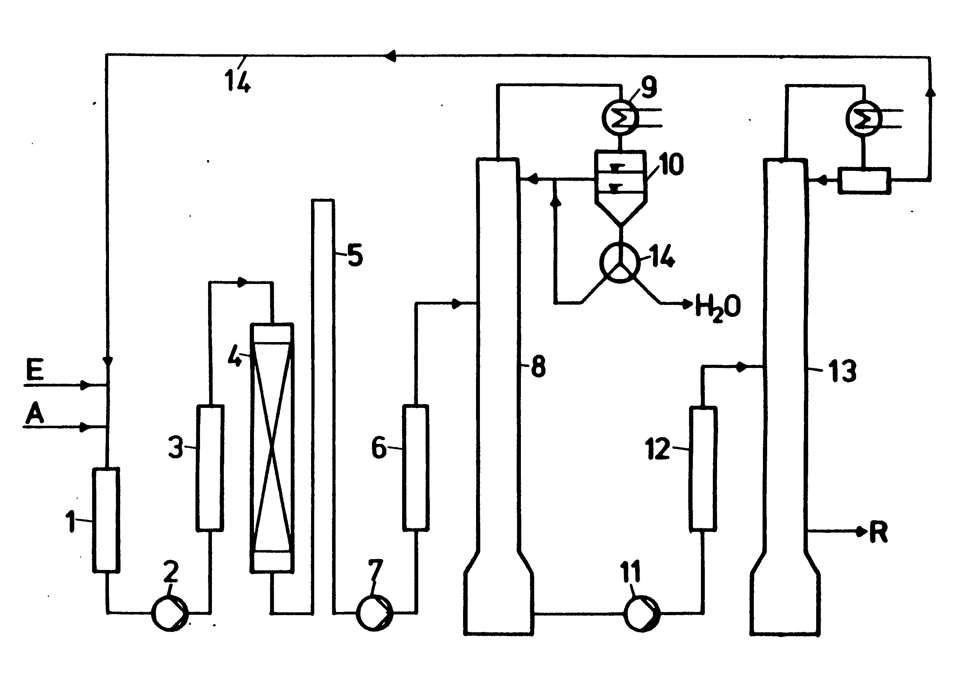

Diethylenglykolmonoethyletheracetat
413 g (6,9 mol) Essigsäure (E in Abb. 1) und 920 g (6,9 mol) Diethylenglykolmonoethylether (A in Abb. 1) werden stündlich mit 567 g Destillat (174 g = 2,9 mol Essigsäure und 393 g = 2,9 mol Diethylenglykolmonoethylether) der Reinesterkolonne (13) in einem statischen Mischer (1) vermischt und mittels einer Pumpe (2) über einen Wärmetauscher (3), in dem das Gemisch auf 80 bis 82°C vorgewärmt wird, dem Reaktor (4) zugeführt Dieser besteht aus einem geraden Rohr (⌀ 80 mm, h - 1 000 mm), das am unteren Ende eine Siebplatte enthält; der Reaktor wird über einen Mantel durch Einsatz einer Wärmeträgerflüssigkeit auf 80 bis 82°C gehalten. Er enthält 1 700 cm$^3$ eines bis zur Volumenkonstanz in die Ethylenglykolmonoethylester gequollenen stark sauren Kationenaustauschers (Polystyrolsulfonsäure mit 18 Gewichtsprozent Divinylbenzol vernetzt, Korngröße 0,3 bis 1,5 mm). Der Reaktor wird bei einem Druck von 1,05 bar geflutet betrieben, wobei die nachgeschaltete hochgezogene Rohrleitung (5) zur Druckhaltung dient. Die Reaktanden reagieren bis zum Gleichgewicht ab, wobei sich 44 bis 48 % des Alkohols zum Ester umsetzen. Das aus dem Reaktor abströmende Gemisch wird über einen Vorwärmer (6) auf 110 bis 115°C aufgeheizt und mittels einer Pumpe (7) auf den 20. Boden einer insgesamt 70 Glockenböden enthaltenden Kolonne (8) gegeben (⌀ 80 mm, Flüssigkeitsholdup des Abtriebsteils einschließlich des Sumpfes 3,0 dm$^3$). Das Reaktionswasser wird bei einer Kopftemperatur von 80°C als Toluol-Wasser-Azeotrop abdestilliert, welches nach Kondensation im Kühler (9) im Phasentrenngefäß (10) in zwei Phasen zerfällt. Die organische, toluolreiche Phase wird vollständig auf den Kolonnenkopf zurückgefahren, die Wasserphase in einer dem Einsatzgemisch entsprechenden Menge abgenommen.

Der Rohester wird flüssig am Sumpf der mittels eines elektrischen Tauchsieders beheizten Kolonne abgezogen (Sumpftemperatur 189 bis 192°C) und mit Hilfe einer Pumpe (11) über einen Vorwärmer (12) in die Reinesterkolonne (13) gegeben.

Der Rohester enthält mit 8 bis 11 Gewichtsprozent Essigsäure und 18 bis 22 Gewichtsprozent Alkohol erheblich weniger Edukte als dem Veresterungsgleichgewicht entspricht. Der Alkoholumsatz, bezogen auf den Reaktorzulauf, beträgt 71 bis 74 %. Die Ausbeute, bezogen auf den eingesetzten Alkohol, beträgt 98,4 bis 98,6 % d. Th.

## Beispiel 2

Ethylenglykolmonoethyletheracetat
Stündlich werden 770 g eines äquimolaren Gemisches (je 5,1 mol) aus Ethylenglykolmonoethylether und Essigsäure mit dem Destillat der Reinesterkolonne (595 g = 6,6 mol Ethylenglykolmonoethylether und 145 g = 2,4 mol Essigsäure) entsprechend dem Beispiel 1 gemischt und dem unter gleichen Bedingungen betriebenen Reaktor, der 1 400 cm³ eines bis zur Volumenkonstanz in Ethylenglykolmonoethylester gequollenen, stark sauren Ionenaustauscher (Polystyrolsulfonsäure mit 8 Gew.-% Divinylbenzol vernetzt, Korngröße 0,3 bis 1,5 mm) enthält, zugeführt. Die Reaktanden reagieren bis zum Gleichgewicht, wobei sich 40 bis 42 % des dem Reaktor zufließenden Alkohols umsetzen. Das aus dem Reaktor abströmende Gemisch wird über einen Vorvärmer (6) auf 94 bis 97°C aufgeheizt und mittels einer Pumpe auf den 25. Boden einer insgesamt 45 Glockenböden enthaltenden Kolonne gegeben (∅ 80 mm, Flüssigkeitsholdup des Abtriebsteils einschließlich des Sumpfes 3,2 l). Das Reaktionswasser wird bei einer Kopftemperatur von 95 bis 96°C mit Ethylenglykolmonoethyletheracetat abdestilliert; die im Phasentrenngefäß (10) anfallende Oberphase wird vollständig auf den Kolonnenkopf zurückgefahren. Die Wasserphase wird mittels eines Flüssigkeitsteilers (14) geteilt; 1 Teil wird als Reaktionswasser ausgeschleust, 2,3 bis 2,5 Teile auf den Kolonnenkopf zurückgegeben.

Der Rohester wird flüssig am Sumpf mit einer Temperatur von 144 bis 146°C abgezogen und wie im Beispiel 1 weiterverarbeitet. Der Rohester enthält 6 bis 8 Gewichtsprozent Essigsäure und 32 bis 36 Gewichtsprozent Alkohol. Der Alkoholumsatz, bezogen auf den Reaktorzulauf, beträgt 56 bis 57 %. Die Ausbeute, bezogen auf den Alkohol, beträgt 98,9 bis 99,3 % d. Th.

### Beispiel 3

2-Ethylhexylacetat

Stündlich werden 1 140 g eines Gemisches (je 6,0 mol) aus 2-Ethylhexanol-(1) und Essigsäure mit dem Destillat der Reinesterkolonne entsprechend dem Beispiel 1 gemischt, dem unter gleichen Bedingungen betriebenen Reaktor zugeführt und in einer Glockenbodenkolonne analog aufgearbeitet.

Der Rohester wird flüssig siedend am Sumpf der Kolonne abgezogen (Sumpftemperatur 184 bis 190°C) und mit Hilfe einer Pumpe (11) über einen Vorwärmboden (12) in die Reinesterkolonne (13) gegeben.

Der Rohester enthält mit 5,0 bis 6,0 Gew.-% Essigsäure und 11 bis 13 Gew.-% Alkohol erheblich weniger Edukte als dem Veresterungsgleichgewicht entspricht. Der Alkoholumsatz, bezogen auf den Reaktorzulauf, beträgt 83 bis 85 %.

Die Ausbeute, bezogen auf den eingesetzten Alkohol, beträgt 99,2 bis 99,7 % d. Th.

## Patentansprüche

1. Verfahren zur Veresterung von Essigsäure mit Alkoholen, deren Essigsäureester höher als Essigsäure sieden, in zwei Stufen in vertikalen Reaktoren und destillativer Trennung des erhaltenen Reaktionsgemisches, wobei man in der ersten Stufe die Veresterung in Gegenwart von feinkörnigen, stark sauren Kationenaustauschern im Ab- oder Aufstrom durchführt, mit Hilfe einer Druckhaltevorrichtung zwischen Reaktor und Destillationskolonne den Reaktorgeflutet betreibt, das den Reaktor verlassende Reaktionsgemisch flüssig in eine Destillationskolonne einführt und das bei der Veresterung anfallende Wasser mit einem Schleppmittel azeotrop austrägt,
dadurch gekennzeichnet,
daß man in einer zweiten Stufe die Veresterung ohne Katalysator im Abtriebsteil und Sumpf einer Kolonne bei Temperaturen oberhalb 100°C und Verweilzeiten von 40 bis 180 min durchführt, den gebildeten Ester gemeinsam mit nicht umgesetztem Alkohol und nicht umgesetzter Essigsäure flüssig aus dem Sumpf der Kolonne abzieht und nach Trennung in einer nachgeschalteten Kolonne die abgetrennten Edukte nach Ergänzung der umgesetzten Anteile in den Reaktor zurückführt.

2. Verfahren nach Anspruch 1,
dadurch gekennzeichnet,
daß man die Veresterung in der zweiten Stufe im Abtriebsteil einer Kolonne durchführt, die dort einen Druckverlust von 2 bis 10 mbar, vorzugsweise 3 bis 8 mbar pro theoretischer Stufe aufweist.

3. Verfahren nach den Ansprüchen 1 und 2,
dadurch gekennzeichnet,
daß man die Veresterung in der zweiten Stufe bei Verweilzeiten von 60 bis 120 min durchführt.

4. Verfahren nach den Ansprüchen 1 bis 3,
dadurch gekennzeichnet,
daß man die Veresterung in der zweiten Stufe in einer Bodenkolonne durchführt.

5. Verfahren nach den Ansprüchen 1 bis 4,
dadurch gekennzeichnet,
daß man das bei der Veresterung anfallende Wasser mit einem im Verfahren erhaltenen Ester, der mit Wasser oder mit Wasser und dem Alkohol ein Heteroazeotrop bildet, als Schleppmittel austrägt.

## Claims

1. A process for esterifying acetic acid using an alcohol whose acetate has a higher boiling point than acetic acid, in two stages in a vertical reactor with distillative separation of the resultant reaction mixture, the esterification in the first stage being carried out in the presence of finegrained, strongly acidic cation exchanger in a downward or upward stream, the reactor being operated in a flooded state with the aid of a pressure-retention apparatus between the reactor and the distillation column, the reaction mixture leaving the reactor being introduced in liquid form into a distillation column, and the water produced during the esterification being removed azeotropically using an entrainer, characterised in that the esterification in the second stage is carried out in the stripping zone and the bottom of a column at a temperature above 100°C and at a residence time from 40 to 180 minutes without catalyst, the ester formed is removed in liquid form from the bottom of the column together with unreacted alcohol and unreacted acetic acid, and, after separation in a downstream column, the educts removed are fed back into a reactor after replenishing the reacted components

2. A process according to claim 1, characterised in that the esterification in the second stage is carried out in the stripping zone of column which at this point has a pressure loss of 2 to 10 mbar, preferably 3 to 8 mbar, per theoretical plate.

3. A process according to any of claims 1 and 2, characterised in that the esterification in the second stage is carried out at a residence time of 60 to 120 minutes.

4. A process according to any of claims 1 to 3, characterised in that the esterification in the second stage is carried out in a tray column.

5. A process according to any of claims 1 to 4, characterised in that the water produced during the esterification is removed using, as entrainer, an ester obtained in the process which forms a heteroazeotrope with water and/or with water and the alcohol.

## Revendications

1. Procédé d'estérification de l'acide acétique avec des alcools dont les esters avec l'acide acétique bouillent plus haut que l'acide acétique, en deux étapes, dans des réacteurs verticaux, et pour la séparation par distillation du mélange réactionnel obtenu, tandis que dans une première étape on effectue l'estérification en courant descendant ou ascendant, en présence d'échangeurs de cations fortement acides se présentant en grains fins, que l'on fait fonctionner le réacteur par arrosage à l'aide d'un dispositif de maintien de pression entre le réacteur et la colonne de distillation, que l'on introduit à l'état liquide dans la colonne de distillation le mélange réactionnel qui quitte le réacteur et que l'on extrait par vole azéotropique avec un agent d'entraînement l'eau qui se forme lors de l'estérification, caractérisé par le fait que l'on effectue dans une seconde étape l'estérification sans catalyseur dans la partie de soutirage et la base d'une colonne à des températures au dessus de 100°C et avec des temps de séjour de 40 à 180 minutes, que l'on soutire à la base de la colonne, à l'état liquide, l'ester formé en commun avec l'alcool non converti et l'acide acétique n'ayant pas réagi, et qu'après séparation dans une colonne montée en aval, on renvoie dans le réacteur les produits séparés qui sortent, après avoir complété les fractions ayant réagi.

2. Procédé selon la revendication 1, caractérisé par le fait que l'on effectue l'estérification dans la deuxième étape dans la partie de soutirage d'une colonne présentant en cet endroit une perte de charge de 2 à 10 m.bars, de préférence de 3 à 8 m.bars par étape théorique.

3. Procédé selon les revendications 1 et 2, caractérisé par le fait que l'on effectue l'estérification dans la deuxième étape avec des temps de séjour de 60 à 120 minutes.

4. Procédé selon les revendications 1 à 3, caractérisé par le fait qu'on effectue l'estérification dans la seconde étape dans une colonne à plateaux.

5. Procédé selon les revendications 1 à 4, caractérisé par le fait que l'on extrait l'eau se formant lors de l'estérification en utilisant, comme agent d'entraînement, un ester obtenu dans le procédé et formant un hétéro-azéotrope avec l'eau ou avec l'eau et l'alcool.

R

$H_2O$

13

12

11

9

10

14

8

6

7

5

4

3

2

1

14

E

A